(19) Europäisches Patentamt
European Patent Office
Office européen des brevets

(11) Veröffentlichungsnummer: **0 284 942**
**A2**

# (12) EUROPÄISCHE PATENTANMELDUNG

(21) Anmeldenummer: **88104483.8**

(22) Anmeldetag: **21.03.88**

(51) Int. Cl.⁴: **C07K 7/06**, A61K 37/02

(30) Priorität: **03.04.87 DE 3711335**

(43) Veröffentlichungstag der Anmeldung:
**05.10.88 Patentblatt 88/40**

(84) Benannte Vertragsstaaten:
**AT BE CH DE ES FR GB IT LI NL SE**

(71) Anmelder: **Merck Patent Gesellschaft mit beschränkter Haftung**
**Frankfurter Strasse 250**
**D-6100 Darmstadt(DE)**

(72) Erfinder: **Weber, Wolf-Dietrich, Dr.**
**Lindenweg 32**
**D-6107 Reinheim 1(DE)**
Erfinder: **Hölzemann, Günter, Dr.**
**Weedring 5**
**D-6104 Seeheim 1(DE)**
Erfinder: **Jonczyk, Alfred, Dr.**
**Eschelkopfweg 5**
**D-6100 Darmstadt(DE)**
Erfinder: **Wild, Albrecht, Prof.Dr.**
**Am Sonderbach 76**
**D-6148 Heppenheim(DE)**
Erfinder: **Lues, Ingeborg, Dr.**
**Paul-Wagner-Strasse 13**
**D-6100 Darmstadt(DE)**
Erfinder: **Wienrich, Marion, Dr.**
**Schumannstrasse 15**
**D-6100 Darmstadt(DE)**
Erfinder: **Greiner, Hartmut, Dr.**
**Dieburgerstrasse 218**
**D-6100 Darmstadt(DE)**

## (54) Aminosäurederivate.

(57) Aminosäurederivate der Formel I

$$X\text{-}Z\text{-}NR^1\text{-}CR^2R^3\text{-}CO\text{-}NR^4\text{-}CHR^5\text{-}CO\text{-}Y\text{-}R^6 \qquad I$$

worin
X,Y,Z und $R^1$ bis $R^6$ die in Patentanspruch 1 angegebene Bedeutung haben,

zeigen tachykinin-agonistische (z.B. vasodilatierende) und/oder tachykininantagonistische (z B. analgetische) Wirkungen.

EP 0 284 942 A2

## Aminosäurederivate

Die Erfindung betrifft neue Aminosäurederivate der Formal I

X-Z-$NR^1$-$CR^2R^3$-CO-$NR^4$-$CHR^5$-CO-Y-$R^6$ I

worin

X H, $R^7$-O-CO-, $R^7$-CO-, $R^7$-NH-CO-oder $R^8$-CO-$(CHR^7)_n$-CO-,

Z 0 bis 4 peptidartig miteinander verbundene Aminosäurereste ausgewählt aus der Gruppe bestehend aus Ala, Arg, Asn, Asp(OBut), Gln, Glu, Gly, His, Ile, Leu, Lys, Met, αNal, βNal, Orn, Phe, Pro, Ser, Thr, Tic, Trp, Tyr, Val, Cα-Me-αNal, Cα-Me-Phe, Cα-Me-Tic, Cα-Me-Trp, Cα-Me-Tyr, wobei funktionelle Gruppen der Aminosäureseitenkette auch mit einer Gruppe X geschützt sein können,

$R^1$, $R^2$, $R^3$, $R^4$, $R^5$, und $R^7$ jeweils H, A, Alkenyl oder Alkinyl mit jeweils bis zu 4 C-Atomen, Ar, Ar-alkyl, Het, Het-alkyl, unsubstituiertes oder ein-oder mehrfach durch A, AO und/oder Hal substituiertes Cycloalkyl mit 3-7 C-Atomen, Cycloalkylalkyl mit 4-11 C-Atomen,

$R^1$ und $R^4$, $R^2$ und $R^3$ bzw. $R^2$ und $R^4$ auch jeweils zusammen eine Alkylenkette mit 2-6 C-Atomen, die gesättigt oder ungesättigt, unsubstituiert oder ein-oder mehrfach durch A, OH, OA, Ar, Ar-alkyl, Het und/oder Het-alkyl substituiert sein kann, wobei $R^2$ und/oder $R^3$ nur dann H sind, wenn $R^1$ und $R^4$ bzw. $R^2$ und $R^4$ jeweils zusammen eine Alkylenkette mit 2-6 C-Atomen, die gesättigt oder ungesättigt, unsubstituiert oder ein-oder mehrfach durch A, OH, OA, Ar, Ar-alkyl und/oder Het-alkyl substituiert sein kann, bedeuten,

Y Cys, Ile, Leu, Met, Met(O), Met($O_2$), Nle oder Phe,

$R^6$ und $R^8$ jeweils OH, OA, $NH_2$, NHA oder $NA_2$,

n 0, 1, 2 oder 3,

Ar unsubstituiertes oder ein-oder mehrfach durch A, AO, Hal, $CF_3$, OH und/oder $NH_2$ substituiertes Phenyl oder unsubstituiertes Naphthyl,

Het einen gesättigten oder ungesättigten 5-oder 6-gliedrigen heterocyclischen Rest mit 1-4 N-, O- und/oder S-Atomen, der mit einem Benzolring oder einem Pyridinring kondensiert und/oder ein-oder mehrfach durch A, AO, Hal, $CF_3$, HO, $O_2N$, Carbonylsauerstoff, $H_2N$, HAN, $A_2N$, AcNH, AS, ASO, $ASO_2$, AOOC, CN, $H_2NCO$, ANHCO, $A_2NCO$, ArHNCO, Ar-alkyl-NHCO, $H_2NSO_2$, $ASO_2NH$, Ar, Ar-alkyl, Ar-alkenyl, Hydroxyalkyl und/oder Aminoalkyl mit jeweils 1-8 C-Atomen substituiert sein kann und/oder dessen N- und/oder S-Heteroatome auch oxydiert sein können,

-alkyl- eine Alkylenkette mit 1-4 C-Atomen,

Hal F, Cl, Br oder J,

Ac H-CO-, A-CO-, Ar-CO-, A-NH-CO-oder Ar-NH-CO-und

A Alkyl mit 1-8 C-Atomen bedeuten,

worin ferner an Stelle einer oder mehrerer -NH-CO-Gruppen auch eine oder mehrere -NA-CO-Gruppen stehen können,

worin jedoch -$NR^1$-$CR^2R^3$-CO-$NR^4$-$CHR^5$-CO-Y-$R^6$ nur dann
(1) Aib-Phe-Met-$NH_2$ bedeutet,
wenn X-Z von BOC-Gly, von H-Tyr und von H-Tyr-Gly verschieden ist;

(2) Aib-Leu-Met-$NH_2$ bedeutet,
wenn X-Z von BOC-Phe-Phe, von BOC-Gln-Phe-Phe, von BOC-Gln-Gln-Phe-Phe und von H-Gln-Gln-Phe-Phe verschieden ist,
(3) Aib-Phe-Leu-$NH_2$ bedeutet,
wenn X-Z von H, von BOC, von H-Tyr und von BOC-Tyr-Gly verschieden ist;
(4) Aib-Phe-Leu-OMe bedeutet,
wenn X-Z von H und von BOC verschieden ist;
(5) Aib-Phe-Phe-OMe bedeutet,
wenn X-Z von CBz verschieden ist;
(6) Aib-Trp-Phe-$NH_2$ bedeutet,
wenn X-Z von H-Tyr-Trp verschieden ist;
(7) Deg-Gly-Leu-OH bedeutet, wenn X-Z von H verschieden ist;

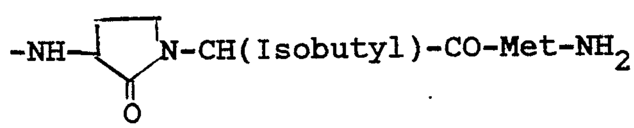

bedeutet,
wenn X-Z von H, von BOC, von Ac-Phe-Ile, von Ac-Phe-Phe, von Ac-Phe-Tyr, von Ac-Phe-Val, von Glp-Phe-Ile, von Glp-Phe-Phe, von Glp-Phe-Tyr, von Glp-Phe-Val, von H-Pro-Phe-Gly, von H-Pro-Phe-Phe, von H-Pro-Trp-Gly, von H-Pro-Trp-Phe, von BOC-Pro-Phe-Phe, von Ac-Ala-Phe-Ile, von Ac-Ala-Phe-Phe, von Ac-Ala-Phe-Tyr, von Ac-Ala-Phe-Val, von Ac-Phe-Phe-Ile, von Ac-Phe-Phe-Phe, von Ac-Phe-Phe-Tyr und von Ac-Phe-Phe-Val verschieden ist;

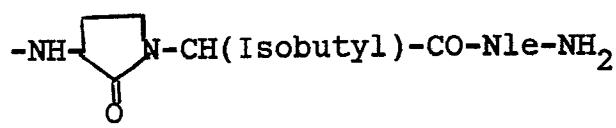

bedeutet,
wenn X-Z von H-Pro-Phe-Gly, von H-Pro-Phe-Phe, von H-Pro-Trp-Gly und von H-Pro-Trp-Phe verschieden ist;

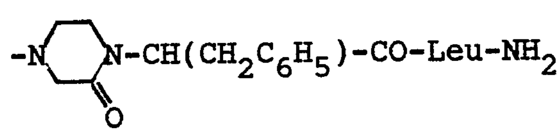

bedeutet,
wenn X-Z von H-Tyr-D-Ala verschieden ist;
(11) Cle-Phe-Leu-$NH_2$ bedeutet,
wenn X-Z von H, von BOC, von H-Tyr-Gly und von BOC-Tyr-Gly verschieden ist;
(12) Cle-Leu-Phe-OH bedeutet,
wenn X-Z von H-CO verschieden ist; sowie deren Salze.

Ähnliche Verbindungen sind aus der EP-A-0176436 und der US-A-4 472 305 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem wirken sie tachykinin-agonistisch (z. B. vasodilatierend) und/oder tachykinin-antagonistisch (z. B. analgetisch). Diese Wirkungen können z. B. nach den Methoden nachgewiesen werden, die in der US-A-4 472 305 angegeben sind, die vasofilatierende Wirkung z. B. auch nach der Methode von F. Lembeck et al., Biochem. Res. Comm. 103, 1318-1321 (1981), die analgetische Wirkung z. B. im Schleiftest an Ratten oder Mäusen (Methodik vgl. C. Vander Wende u. S. Margolin, Fed. Proc. 15, 494 ff. (1956); E. Siegmund et al., Proc. Soc. exp. Biol. (NY) 95, 729-731 (1957); L.L. Hendershot u. J. Forsaith, J. Pharmacol. exp. Ther. 125, 237-240 (1959)). Bei den Agonisten treten weiterhin Anregungen der Motorik und Blutdrucksenkungen, bei den Antagonisten antiinflamma torische und/oder spasmolytische Effekte auf. Weiterhin zeigen sich bei den Verbindungen der Formel I stimulierende Wirkungen auf die Tränensekretion, insbesondere bei lokaler Anwendung.

Die Verbindungen können als Arzneimittelwirkstoffe in der Human-und Veterinärmedizin eingesetzt werden, insbesondere zur Prophylaxe und zur Behandlung von cardiovaskulären Erkrankungen, spastischen Erkrankungen, Schmerzzuständen, Entzündungen, Erkrankungen des Zentralnervensystems und/oder des Kreislaufs, und/oder zur Stimulierung der Tränensekretion.

Die vor-und nachstehend aufgeführten Abkürzungen von Aminosäureresten stehen für die Reste -NH-CR'R"-CO-(worin R, R' und R" die für jede Aminosäure bekannten spezifischen Bedeutungen haben) folgender Aminosäuren:

Aib 2-Amino-isobuttersäure
Ala Alanin
Arg Arginin
Asn Asparagin
Asp Asparaginsäure
Asp(OBut) Asparaginsäure-mono-tert.-butylester
Cle "Cyclo-leucin" (1-Amino-cyclopentancarbonsäure)
Cα-Me-αNal C-α-Methyl-3-(α-naphthyl)-alanin
Cα-Me-Phe C-α-Methyl-phenylalanin
Cα-Me-Tic 1-Methyl-1,2,3,4-tetrahydroisochinolin-1-carbonsäure
Cα-Me-Trp C-α-Methyl-tryptophan
Cα-Me-Tyr C-α-Methyl-tyrosin
Cys Cystein
Deg Diethylglycin(2-Amino-2-ethyl-buttersäure)
Dpg Dipropylglycin(2-Amino-2-propylvaleriansäure)
Gln Glutamin
Glu Glutaminsäure
Gly Glycin
His Histidin
Ile Isoleucin
Leu Leucin
Lys Lysin
Lys(CBZ) $N^5$-Benzyloxycarbonyl-lysin
Met Methionin
Met(O) Methionin-S-oxid
Met($O_2$) Methionin-S,S-dioxid
αNal 3-(α-Naphthyl)-alanin
βNal 3-(β-Naphthyl)-alanin
Nle Norleucin
N-Me-Phe N-Methyl-phenylalanin
N-Me-Trp N-Methyl-tryptophan
Orn Ornithin
Phe Phenylalanin
Pro Prolin
Ser Serin
Thr Threonin
Tic 1,2,3,4-Tetrahydroisochinolin-1-carbonsäure
Trp Tryptophan
Tyr Tyrosin
Val Valin.

Ferner bedeutet nachstehend

BOC tert.-Butoxycarbonyl
imi-BOM Benzyloxymethyl in 1-Stellung des Imidazolrings
CBZ Benzyloxycarbonyl
DCCI Dicyclohexylcarbodiimid
DMF Dimethylformamid
DNP 2,4-Dinitrophenyl

imi-DNP 2,4-Dinitrophenyl in 1-Stellung des Imidazolrings
EDCI N-Ethyl-N'-(3-dimethylaminopropyl)-carbodiimidhydrochlorid
ETNC N-Ethylcarbamoyl
ETOC Ethoxycarbonyl
FMOC 9-Fluorenylmethoxycarbonyl
Glp Pyroglutamyl (5-Oxopyrrolidyl-2-carbonyl)
HOBt 1-Hydroxybenzotriazol
IPNC N-Isopropylcarbamoyl
IPOC Isopropoxycarbonyl
MC Morpholinocarbonyl
Me Methyl
OMe Methylester
OEt Ethylester
OSu Ester mit N-Hydroxysuccinimid
PBB 4-Phenyl-2-benzylbutyryl
POA Phenoxyacetyl
Succ Succinyl (3-Carboxypropionyl)
TFA Trifluoressigsäure
Tfa Trifluoracetat

Sofern die vorstehend genannten Aminosäuren in mehreren enantiomeren Formen auftreten können, so sind vor-und nachstehend, z. B. als Bestandteil der Verbindungen der Formel I, alle diese Formen und auch ihre Gemische (z. B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich vas anderes angegeben ist.

Gegenstand der Erfindung ist ferner ein Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

$X\text{-}G^1\text{-}OH$ II

worin $G^1$

(a) $-Z^1-$,
(b) $-Z-$,
(c) $-Z\text{-}NR^1\text{-}CR^2R^3\text{-}CO-$,
(d) $-Z\text{-}NR^1\text{-}CR^2R^3\text{-}CO\text{-}NR^4\text{-}CHR^5\text{-}CO-$,
(e) $-Z\text{-}NR^1\text{-}CR^3R^3\text{-}CO\text{-}NR^4\text{-}CHR^5\text{-}CO\text{-}Y$-bedeutet

mit einer Aminoverbindung der Formel III

$H\text{-}G^2$ III

worin $G^2$

(a) $-Z^2\text{-}NR^1\text{-}CR^2R^3\text{-}CO\text{-}NR^4\text{-}CHR^5\text{-}CO\text{-}Y\text{-}R^6$,
(b) $-NR^1\text{-}CR^2R^3\text{-}CO\text{-}NR^4\text{-}CHR^5\text{-}CO\text{-}Y\text{-}R^6$,
(c) $-NR^4\text{-}CHR^5\text{-}CO\text{-}Y\text{-}R^6$,
(d) $-Y\text{-}R^6$,
(e) $-NH_2$, NHA oder $NA_2$ und $Z^1 + Z^2$ zusammen Z bedeuten

umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Aminogruppe acyliert und/oder einen Rest $R^6$ und/oder $R^8$ durch Behandeln mit veresternden, solvolysierenden oder amidierenden Mitteln in einen anderen Rest $R^6$ und/oder $R^8$ umwandelt und/ oder eine Thioether gruppe zu einer Sulfoxidgruppe oder Sulfongruppe

oxydiert und/oder eine Sulfoxidgruppe zu einer Thioethergruppe reduziert und/oder eine Verbindung der Formel I durch Behandeln mit einer Säure in eines ihrer Salze überführt.

Vor-und nachstehend haben die Reste bzw. Parameter X, Y, Z, $R^1$ bis $R^8$, n, Ar, Het, Hal, Ac, A, $G^1$, $G^2$, $Z^1$ und $Z^2$ die bei den Formeln I, II oder III angegebenen Bedeutungen, falls nicht ausdrücklich etwas anderes angegeben ist. Falls zwei Reste $R^7$ oder $R^8$ in einer Verbindung der Formel I vorhanden sind, können sie gleich oder voneinander verschieden sein.

In den vorstehenden Formeln hat A 1 - 8, vorzugsweise 1,2,3 oder 4 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2-oder 3-Methylbutyl, 1,1-, 1,2-oder 2,2-Dimethylpropyl, 1-Ethylpropyl, Hexyl, 1-, 2-, 3-oder 4-Methylpentyl, 1,1-, 1,2-, 1,3-, 2,2-, 2,3-oder 3,3-Dimethylbutyl, 1-oder 2-Ethylbutyl, 1-Ethyl-1-methylpropyl, 1-Ethyl2-methylpropyl, 1,1,2-oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z. B. 1-, 2-oder 3-Methylcyclopentyl, 1-, 2-, 3-oder 4-Methylcyclohexyl.

Dementsprechend bedeutet Cycloalkyl-alkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z. B. 1-, 2-oder 3-Methylcyclopentylmethyl, 1-, 2-, 3-oder 4-Methylcyclohexylmethyl.

Hal bedeutet vorzugsweise F, Cl oder Br, aber auch J.

Ac bedeutet vorzugsweise H-CO-, A-CO-wie Acetyl, Propionyl oder Butyryl, Ar-CO-wie Benzoyl, o-, m-oder p-Methoxybenzoyl oder 3,4-Dimethoxybenzoyl, A-NH-CO-wie N-Methyl-oder N-Ethylcariamoyl, Ar-NH-CO-wie N-Phenylcarbamoyl.

Ar bedeutet vorzugsweise Phenyl, ferner bevorzugt o-, m-oder p-Tolyl, o-, m-oder p-Ethylphenyl, o-, m-oder p-Methoxyphenyl, o-, m-oder p-Fluorphenyl, o-, m-oder p-Chlorphenyl, o-, m-oder p-Bromphenyl, o-, m-oder p-Jodphenyl, o-, m-oder p-Trifluormethylphenyl, o-, m-oder p-Hydroxyphenyl, 2,3-, 2,4-, 2,5-, 2,6-, 3,4-oder 3,5-Dimethoxyphenyl, 3,4,5-Trimethoxyphenyl, o-, m-oder p-Aminophenyl, 1-oder 2-Naphthyl.

Dementsprechend bedeutet Ar-alkyl vorzugsweise Benzyl, 1-oder 2-Phenylethyl, o-, m-oder p-Methylbenzyl, 1-oder 2-o-, -m-oder -p-Tolylethyl, o-, m-oder p-Ethylbenzyl, 1-oder 2-o-, -m-oder -p-Ethylphenylethyl, o-, m-oder p-Methoxybenzyl, 1-oder 2-o-, -m-oder -p-Methoxyphenylethyl, o-, m-oder p-Fluorbenzyl, 1-oder 2-o-, -m-oder -p-Fluorphenylethyl, o-, m-oder p-Chlorbenzyl, 1-oder 2-o-, -m-oder -p-Chlorphenylethyl, o-, m-oder p-Brombenzyl, 1-oder 2-o-, -m-oder -p-Bromphenylethyl, o-, m-oder p-Jodbenzyl, 1-oder 2-o-, -m-oder -p-Jodphenylethyl, o-, m-oder p-Trifluormethylbenzyl, o-, m-oder p-Hydroxybenzyl, 2,3-, 2,4-, 2,5-, 2,6-, 3,4-oder 3,5-Dimethoxybenzyl, 3,4,5-Trimethoxybenzyl, o-, m-oder p-Aminobenzyl, 1-oder 2-Naphthylmethyl.

Het ist vorzugsweise 2-oder 3-Furyl, 2-oder 3-Thienyl, 1-, 2-oder 3-Pyrryl, 1-, 2-, 4-oder 5-Imidazolyl, 1-, 3-, 4-oder 5-Pyrazolyl, 2-, 4-oder 5-Oxazolyl, 3-, 4-oder 5-Isoxazolyl, 2-, 4-oder 5-Thiazolyl, 3-, 4-oder 5-Isothiazolyl, 2-, 3-oder 4-Pyridyl, 2-, 4-, 5-oder 6-Pyrimidyl, weiterhin bevorzugt 1,2,3-Triazol-1-, -4-oder -5-yl, 1,3,4-Triazol-1-, -3-oder -5-yl, 1-oder 5-Tetrazolyl, 1,2,3-Oxadiazol-4-oder -5-yl, 1,2,4-Oxadiazol-3-oder -5-yl, 1,3,4-Thiadiazol-2-oder -5-yl, 1,2,4-Thiadiazol-3-oder -5-yl, 2,1,5-Thiadiazol-3-oder -4-yl, 2-, 3-, 4-, 5-oder 6-2H-Thiopyranyl, 2-, 3-oder 4-4H-Thiopyranyl, 3-oder 4-Pyridazinyl, Pyrazinyl, 2-, 3-, 4-, 5-, 6-oder 7-Benzofuryl, 2-, 3-, 4-, 5-, 6-oder 7-Benzothienyl, 1-, 2-, 3-, 4-, 5-, 6-oder 7-Indolyl, 1-, 2-, 3-, 4-, 5-, 6-oder 7-Isoindolyl, 1-, 2-, 4-oder 5-Benzimidazolyl, 1-, 3-, 4-, 5-, 6-oder 7-Benzopyrazolyl, 2-, 4-, 5-, 6-oder 7-Benzooxazolyl, 3-, 4-, 5-, 6-, 7-Benzisoxazolyl, 2-, 4-, 5-, 6-oder 7-Benzthiazolyl, 2-, 4-, 5-, 6-oder 7-Benzisothiazolyl, 4-, 5-, 6-oder 7-Benz-2,1,3-oxadiazolyl, 2-, 3-, 4-, 5-, 6-, 7-, oder 8-Chinolyl, 1-, 3-, 4-, 5-, 6-, 7-oder 8-Isochinolyl, 1-, 2-, 3-, 4-oder 9-Carbazolyl, 1-, 2-, 3-, 4-, 5-, 6-, 7-, 8-oder 9-Acridinyl, 3-, 4-, 5-, 6-, 7-oder 8-Cinnolyl, 2-, 4-, 5-, 6-, 7-oder 8-Chinazolyl. Die heterocyclischen Reste können auch teilweise oder vollständig hydriert sein. Het kann also z. B. auch bedeuten 2,3-Dihydro-2-, -3-, -4-oder -5-furyl, 2,5-Dihydro-2-, -3-, -4-oder 5-furyl, Tetrahydro-2-oder -3-furyl, Tetrahydro-2-oder -3-thienyl, 2,3-dihydro-1-, -2-, -3-, -4-oder -5-pyrryl, 2,5-Dihydro-1-, -2-, -3-, -4-oder -5-pyrryl, 1-, 2-oder 3-Pyrrolidinyl, Tetrahydro-1-, -2-oder -4-imidazolyl, 2,3-Dihydro-1-, -2-, -3-, -4-oder 5-pyrazolyl, 2,5-Dihydro-1-, -2-, -3-, -4-oder 5-pyrazolyl, Tetrahydro-1-, -3-, oder -4-pyrazolyl, 1,4-Dihydro-1-, -2-, -3-oder -4-pyridyl, 1,2,3,4-Tetrahydro-1-, -2-, -3-, -4-, -5-oder -6-pyridyl, 1,2,3,6-Tetrahydro-1-, -2-, -3-, -4-, -5-oder -6-pyridyl, 1-, 2-, 3-oder 4-Piperidinyl, 2-, 3-oder 4-Morpholinyl, Tetrahydro-2-, -3-oder -4-pyranyl, 1,4-Dioxanyl, 1,3-Dioxan-2-, -4-oder -5-yl, Hexahydro-1-, -3-oder -4-pyridazinyl, Hexahydro-1-, -2-, -4-oder -5-pyrimidyl, 1-, 2-oder 3-Piperazinyl, 1,2,3,4-Tetrahydro-1-, -2-, -3-, -4-, -5-, -6-, -7-, oder -8-chinolyl, 1,2,3,4-Tetrahydro-1-, -2-, -3-, -4-, -5-, -6-, -7-oder 8-isochinolyl.

Die heterocyclischen Reste können auch wie angegeben substituiert sein. Het kann also bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 2-Amino-5,6-dimethyl-3-pyrazinyl, 4-Carbamoylpiperidino, ferner z. B. 3-, 4-oder 5-Methyl-2-furyl, 2-, 4-oder 5-Methyl-3-furyl, 2,4-Dimethyl-3-furyl, 5-Nitro-2-furyl, 5-Styryl-2-furyl, 3-, 4-oder 5-Methyl-2-thienyl,

2-, 4-oder 5-Methyl-3-thienyl, 3-Methyl-5-tert.-butyl-2-thienyl, 5-Chlor-2-thienyl, 5-Phenyl-2-oder -3-thienyl, 1-, 3-, 4-oder 5-Methyl-2-pyrryl, 1-Methyl-4-oder -5-nitro-2-pyrryl, 3,5-Dimethyl-4-ethyl-2-pyrryl, 4-Methyl-5-pyrazolyl, 4-oder 5-Methyl-2-thiazolyl, 2-oder 5-Methyl-4-thiazolyl, 2-oder 4-Methyl-5-thiazolyl, 2,4-Dimethyl-5-thiazolyl, 3-, 4-, 5-oder 6-Methyl-2-pyridyl, 2-, 4-, 5-oder 6-Methyl-3-pyridyl, 2-oder 3-Methyl-4-pyridyl, 3-, 4-, 5-oder 6-Chlor-2-pyridyl, 2-, 4-, 5-oder 6-Chlor-3-pyridyl, 2-oder 3-Chlor-4-pyridyl, 2,6-Dichlorpyridyl, 2-Hydroxy-3-, -4-, -5-oder -6-pyridyl (= 1H-2-Pyridon-3-, -4-, -5-oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4-pyridon-5-yl, 5-oder 6-Methyl-4-pyrimidyl, 2,6-Dihydroxy-4-pyrimidyl, 5-Chlor-2-methyl-4-pyrimidyl, 2-Methyl-4-amino-5-pyrimidyl, 3-Methyl-2-benzofuryl, 2-Ethyl-3-benzofuryl, 7-Methyl-2-benzothienyl, 1-, 2-, 4-, 5-, 6-oder 7-Methyl-3-indolyl, 1-Methyl-5-oder -6-benzimidazolyl, 1-Ethyl-5-oder -6-benzimidazolyl, 3-, 4-, 5-, 6-, 7-oder 8-Hydroxy-2-chinolyl.

$R^1$ und $R^4$ bedeuten vorzugsweise jeweils H oder zusammen eine Alkylenkette mit 2-6, insbesondere 2 C-Atomen, in diesem Fall sind $R^2$ und $R^3$ bevorzugt jeweils H.

$R^2$ und $R^3$ sind sonst bevorzugt gleich und bedeuten vorzugsweise jeweils A, insbesondere Methyl, ferner Ethyl, Propyl, Isopropyl, Butyl oder Isobutyl. Ferner bedeuten $R^2$ und $R^3$ bevorzugt zusammen eine Alkylenkette mit 2-6, insbesondere 4 C-Atomen.

Weiterhin bedeuten $R^2$ und $R^4$ vorzugsweise zusammen eine Alkylenkette mit 2-6, insbesondere 2 oder auch 3, C-Atomen; in diesem Fall ist $R^3$ vorzugsweise H.

Dementsprechend ist die Gruppe $-NR^1-CR^2R^3-CO-NR^4-$vorzugsweise $-NH-C(A)_2-CO-NH-$, insbesondere $-NH-C(CH_3)_2-CO-NH-$(= Aib-NH-), ferner $-NH-C(C_2H_5)_2-CO-NH-$(= Deg-NH-) oder $-NH-C(C_3H_7)_2-CO-NH-$(= -Dpg-NH-);

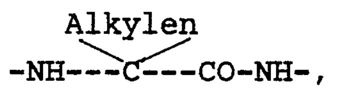, insbesondere 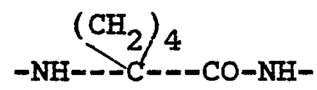

(= -Cle-NH-);

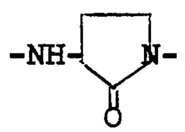; 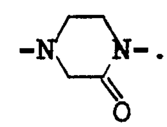.

$R^5$ ist vorzugsweise H oder A, insbesondere Alkyl mit 1-4 C-Atomen, besonders bevorzugt Isobutyl, ferner bevorzugt n-Butyl, sek.-Butyl, Isopropyl.

$R^6$ ist bevorzugt $NH_2$, ferner bevorzugt OMe oder OEt.

Y ist bevorzugt Met, ferner bevorzugt Ile, Leu, Nle oder Phe.

$R^7$ ist bevorzugt H, A, Ar, Ar-alkyl oder 5-Oxo-pyrrolidin-2-yl (= Glp), insbesondere H, Alkyl mit 1-4 C-Atomen, Phenyl, Benzyl oder Glp.

$R^8$ ist bevorzugt OH, ferner bevorzugt OMe oder OEt.

X bedeutet vorzugsweise H, POA, Alkoxycarbonyl wie ETOC, IPOC oder BOC, CBZ, Alkanoyl wie Acetyl, Propionyl, Butyryl oder Isobutyryl, Cycloalkylcarbonyl wie Cyclopentylcarbonyl oder Cyclohexylcarbonyl, Aroyl wie Benzoyl, Arylalkanoyl wie Phenylacetyl, 2-oder 3-Phenylpropionyl, 4-Phenylbutyryl, 2-Benzyl-3-phenylpropionyl, PBB, 2-(2-Phenylethyl)-4-phenylbutyryl, 2-(1-Naphthylmethyl)-4-phenylbutyryl, 2-oder 3-o-, -m-oder -p-Fluorphenylpropionyl, 2-oder 3-o-, -m-oder -p-Chlorphenylpropionyl, Cycloalkylalkanoyl wie Cyclohexylacetyl, 2-oder 3-Cyclohexylpropionyl, N-Alkylcarbamoyl wie ETNC oder IPNC, MC. Besonders bevorzugte Reste X sind H, BOC, CBZ, Glp und Succ, ferner ETOC, IPOC und MC.

Z bedeutet vorzugsweise 2, aber auch 3 oder 4, weiterhin 0 oder 1 peptidartig miteinander verbundene Aminosäurereste, insbesondere eine der Gruppen Phe-Phe, Phe-D-Phe, Lys(CBZ)-Phe-Phe, Lys-Phe-Phe, Lys(CBZ)-Phe-D-Phe, Lys-Phe-D-Phe, Lys(CBZ)-D-Phe-Phe, Lys-D-Phe-Phe, Lys(CBZ)-D-Phe-D-Phe, Lys-D-Phe-D-Phe, Lys(CBZ)-Trp-Phe, Lys-Trp-Phe, Lys(CBZ)-Trp-D-Phe, Lys-Trp-D-Phe, Lys(CBZ)-D-Trp-Phe, Lys-D-Trp-Phe, Lys(CBZ)-D-Trp-D-Phe, Lys-D-Trp-D-Phe, Asp-Phe-Phe-Val, ferner bevorzugt eine der Gruppen N-Me-Trp-Phe, N-Me-Trp-D-Phe, Trp-Phe, Trp-D-Phe, D-Trp-Phe, D-Trp-D-Phe, Asn-Trp-Phe, Asn-Trp-D-Phe, Asn-D-Trp-Phe, Asn-D-Trp-D-Phe, Gln-Trp-Phe, Gln-Trp-D-Phe, Gln-D-Trp-Phe, Gln-D-Trp-D-Phe, Lys(CBZ)-CαMe-Trp-Phe, Lys-CαMe-Trp-Phe, Lys(CBZ)-CαMe-Trp-D-Phe, Lys-CαMe-Trp-D-Phe, Lys(CBZ)-D-CαMe-Trp-Phe, Lys-D-CαMe-Trp-Phe, Lys(CBZ)-D-CαMe-Trp-D-Phe, Lys-D-CαMe-Trp-D-Phe, Lys(CBZ)-N-Me-Trp-Phe, Lys-N-Me-Trp-Phe, Lys(CBZ)-N-Me-Trp-D-Phe, Lys-N-Me-Trp-D-Phe, Lys(CBZ)-D-N-Me-Trp-Phe, Lys-D-N-Me-Trp-Phe, Lys(CBZ)-D-N-Me-Trp-D-Phe, Lys-D-N-Me-Trp-D-Phe, Lys(CBZ)-αNal-Phe, Lys-αNal-Phe, Lys(CBZ)-αNal-D-Phe, Lys-αNal-D-Phe, Lys(CBZ)-D-αNal-Phe, Lys-D-αNal-Phe,

Lys(CBZ)-DαNal-D-Phe, Lys-D-αNal-D-Phe, Lys(CBZ)-Trp-Ile, Lys-Trp-Ile, Lys(CBZ)-Trp-D-Ile, Lys-Trp-D-Ile, Lys(CBZ)-D-Trp-Ile, Lys-D-Trp-Ile, Lys(CBZ)-D-Trp-D-Ile, Lys-D-Trp-D-Ile, Lys(CBZ)-Trp-Leu, Lys-Trp-Leu, Lys(CBZ)-Trp-D-Leu, Lys-Trp-D-Leu, Lys(CBZ)-D-Trp-Leu, Lys-D-Trp-Leu, Lys(CBZ)-D-Trp-D-Leu, Lys-D-Trp-D-Leu, Lys(CBZ)-Trp-N-Me-Phe, Lys-Trp-N-Me-Phe, Lys(CBZ)-Trp-D-N-Me-Phe, Lys-Trp-D-N-Me-Phe, Lys(CBZ)-D-Trp-N-Me-Phe, Lys-D-Trp-N-Me-Phe, Lys(CBZ)-D-Trp-D-N-Me-Phe, Lys-D-Trp-D-N-Me-Phe, Lys(CBZ)-Trp-Trp, Lys-Trp-Trp, Lys(CBZ-Trp-D-Trp, Lys-Trp-D-Trp, Lys(CBZ)-D-Trp-Trp, Lys-D-Trp-Trp, Lys(CBZ)-D-Trp-D-Trp, Lys-D-Trp-D-Trp, Lys(CBZ)-Trp-Val, Lys-Trp-Val, Lys(CBZ)-Trp-D-Val, Lys-Trp-D-Val, Lys(CBZ)-D-Trp-Val, Lys-D-Trp-Val, Lys(CBZ)-D-Trp-D-Val, Lys-D-Trp-D-Val, Lys(CBZ)-Tyr-Phe, Lys-Tyr-Phe, Lys(CBZ)-Tyr-D-Phe, Lys-Tyr-D-Phe, Lys(CBZ)-D-Tyr-Phe, Lys-D-Tyr-Phe, Lys(CBZ)-D-Tyr-D-Phe, Lys-D-Tyr-D-Phe, Orn(CBZ)-Trp-Phe, Orn-Trp-Phe, Orn(CBZ)-Trp-D-Phe, Orn-Trp-D-Phe, Orn(CBZ)-D-Trp-Phe, Orn-D-Trp-Phe, Orn(CBZ)-D-Trp-D-Phe, Orn-D-Trp-D-Phe, Phe-Phe-Val, Phe-Phe-D-Val, Phe-D-Phe-Val, Phe-D-Phe-D-Val, D-Phe-Phe-Val, D-Phe-Phe-D-Val, D-Phe-D-Phe-Val, D-Phe-D-Phe-D-Val, Pro-Trp-Phe, Pro-Trp-D-Phe, Pro-D-Trp-Phe, Pro-D-Trp-D-Phe, D-Pro-Trp-Phe, D-Pro-Trp-D-Phe, D-Pro-D-Trp-Phe, D-Pro-D-Trp-D-Phe.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis If ausgedrückt werden, die der Formel I entsprechen, worin jedoch die Gruppe

$-NR^1-CR^2R^3-CO-NR^4$

in Ia $-NH-C(A)_2-CO-NH-$ bedeutet;

in Ib -Aib-NH-, -Deg-NH- oder -Dpg-NH- bedeutet;

in Ic 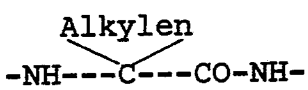 bedeutet;

In Id -Cle-NH- bedeutet;

in Ie 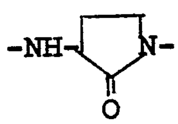 bedeutet;

in If 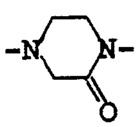 bedeutet.

Besonders bevorzugt sind Verbindungen der Formeln I' sowie Ia' bis If', die den Formeln I sowie Ia bis If entsprechend, worin aber zusätzlich

$R^5$ H oder A und/oder

Y Met, Ile, Leu, Nle oder Phe und/oder

$R^6$ $NH_2$, OMe oder OEt bedeuten,

sowie Verbindungen der Formeln I" sowie Ia" bis If", die den Formeln I sowie Ia bis If entsprechen, worin aber zusätzlich

$R^5$ Isobutyl und/oder

Y Met und/oder

$R^6$ $NH_2$ oder OMe bedeuten.

Insbesondere bevorzugt sind unter den Verbindunden der Formeln I, I', I", Ia bis If, Ia' bis If' sowie Ia" bis If" diejenigen, in denen

X H, BOC, CBZ oder Glp und/oder in denen

Z Phe-Phe, Phe-D-Phe, Lys(CBZ)-D-Trp-D-Phe oder Asp-Phe-Phe-Val bedeuten.

Die Verbindungen der Formel I und auch die Ausgangsstoffe zu ihrer Herstellung werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; vgl. ferner EP-A-0176436 und US-A-4 472 305) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino-und/oder Hydroxygruppen entsprechende geschützte Amino-und/oder Hydroxygruppen enthalten, vorzugsweise solche, die an Stelle eines H-Atoms, das mit einem N-Atom verbunden ist, eine Aminoschutzgruppe tragen, z. B. solche, die der Formel I entsprechen, aber an Stelle einer His-Gruppe eine N(im)-$R^9$-His-Gruppe (worin $R^9$ eine Aminoschutzgruppe bedeutet, z. B. BOM oder DNP) enthalten.

Ferner sind Ausgangsstoffe bevorzugt, die an Stelle des H-Atoms einer Hydroxygruppe eine Hydroxyschutzgruppe tragen, z. B. solche, die der Formel I entsprechen, aber anstelle einer Ser-oder Asp-Gruppe eine -NH-CH($CH_2OR^{10}$)-CO-oder -NH-CH($CH_2COOR^{10}$)-CO-Gruppe enthalten (worin $R^{10}$ eine Hydroxyschutzgruppe bedeutet).

Es können auch mehrere - gleiche oder verschiedene - geschützte Amino-und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-(z. B. DNP), Aralkoxymethyl-(z. B. BOM) oder Aralkylgruppen (z. B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl-und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-Jodethoxycarbonyl; Aralkyloxycarbonyl wie CBZ ("Carbobenzoxy"), 4-Methoxybenzyloxycarbonyl, FMOC. Bevorzugte Aminoschutzgruppen sind DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl-oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure-und Peptidsynthese hergestellt werden, wie sie z. B. in den

genannten Standardwerken und Patentanmeldungen beschrieben sind, z. B. auch nach der Festphasenmethode nach Merrifield.

Das In-Freiheit-Setzen der Verbindungen der Formel I aus ihren funktionellen Derivaten gelingt - je nach der benutzten Schutzgruppe - z. B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol-oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z. B. bevorzugt mit 40%iger Trifluoressigsäure in Methylenchlorid oder mit etwa 3 bis 5 n HCl in Dioxan bei 15-30° abgespalten werden, die FMOC-Gruppe mit einer etwa 5-bis 20%igen Lösung von Dimethylamin, Diethylamin oder Piperidin in DMF bei 15-30°. Eine Abspaltung der DNP-Gruppe gelingt z.B. auch mit einer etwa 3-bis 10%igen Lösung von 2-Mercaptoethanol in DMF/Wasser bei 15-30°.

Hydrogenolytisch entfernbare Schutzgruppen (z. B. BOM, CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5-bis 10%igem Pd-C in Methanol oder mit Ammoniumformiat (an Stelle von $H_2$) und Pd-C in Methanol/DMFbei 20-30°.

Verbindungen der Formel I können auch durch direkte Peptidsynthese aus einer Carbonsäure-(Formel II) und einer Aminkomponente (Formel III) erhalten werden. Als Carbonsäurekomponenten eignen sich z. B. solche der Teilformeln X-Z-OH, X-Z-$NR^1$-$CR^2R^3$-COOH, X-Z-$NR^1$-$CR^2R^3$-CO-$NR^4$-$CHR^5$-COOH oder X-Z-$NR^1$-$CR^2R^3$-CO-$NR^4$-$CHR^5$-CO-Y-OH, als Aminkomponenten solche der Teilformeln H-$NR^1$-$CR^2R^3$-CO-$NR^4$-$CHR^5$-CO-Y-$R^6$, H-$NR^4$-$CHR^5$-CO-Y-$R^6$, H-Y-$R^6$ oder $HR^6$ (worin $R^6$ $NH_2$, NHA oder $NA_2$ bedeutet). Die Peptidbindung kann aber auch innerhalb der Gruppe Z geknüpft werden; dabei wird eine Carbonsäure der Formel X-$Z^1$-OH mit einer Aminoverbindung der Formel H-$Z^2$-$NR^1$-$CR^3R^3$-CO-$NR^4$-$CHR^5$-CO-Y-$R^6$ umgesetzt, wobei $Z^1 + Z^2 = Z$ ist. Dabei arbeitet man zweckmäßig nach üblichen Methoden der Peptid-Synthese, wie sie z. B. in Houben-Weyl, 1.c., Band 15/II, Seiten 1 bis 806 (1974) beschrieben sind.

Die Reaktion gelingt vorzugsweise in Gegenwart eines Dehydratisierungsmittels, z. B. eines Carbodiimids wie DCCI oder EDCI, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, oder in Gemischen dieser Lösungsmittel, bei Temperaturen zwischen etwa -10 und 40, vorzugsweise zwischen 0 und 30°.

Anstelle von II bzw. III können auch geeignete reaktionsfähige Derivate dieser Stoffe in die Reaktion eingesetzt werden, z. B. solche, in denen reaktive Gruppen intermediär durch Schutzgruppen blockiert sind. Die Aminosäurederivate III können z. B. in Form ihrer aktivierten Ester verwendet werden, die zweckmäßig in situ gebildet werden, z. B. durch Zusatz von HOBt oder N-Hydroxysuccinimid.

Die Ausgangsstoffe der Formeln II und III sind großenteils bekannt. Sofern sie nicht bekannt sind, können sie nach bekannten Methoden, z. B. den oben angegebenen Methoden der Peptidsynthese und der Abspaltung von Schutzgruppen, hergestellt werden.

Gewünschtenfalls kann in einer Verbindung der Formel I eine funktionell abgewandelte Amino-und/oder Hydroxygruppe durch Solvolyse oder Hydrogenolyse nach einer der oben beschriebenen Methoden in Freiheit gesetzt werden.

So kann insbesondere eine Verbindung der Formel I, worin X von H verschieden ist, in eine Verbindung der Formel I (X = H) umgewandelt werden, zweckmäßig durch Hydrogenolyse, falls X = CBZ ist, sonst durch selektive Solvolyse. Falls X = BOC ist, kann man z. B. mit HCl in Dioxan bei Raumtemperatur die BOC-Gruppe abspalten. Ferner kann man z. B. Verbindungen, die eine Lys(CBZ)-Gruppe enthalten, in

entsprechenden Verbindungen umwandeln, die stattdessen eine Lys-Gruppe enthalten.

Ferner kann man eine freie Aminogruppe (z. B. in einer Verbindung der Formel I, X = H) in üblicher Weise acylieren, indem man sie mit einer Säure der Formel X-OH (worin X die angegebene Bedeutung hat, aber von H verschieden ist) oder einem reaktionsfähigen Derivat einer solchen Säure umsetzt. Als reaktionsfähige Derivate eignen sich z. B. die Chloride (z. B. Acetylchlorid, CBZ-chlorid), die Bromide (z. B. Benzoylbromid) oder die Anhydride (z. B. Acetanhydrid, Bernsteinsäureanhydrid) der genannten Säuren. Man acyliert zweckmäßig in einem der genannten inerten Lösungsmittel, wobei der Zusatz einer Base wie Triethylamin oder Pyridin vorteilhaft sein kann.

Weiterhin ist es möglich, einen Rest $R^6$ und/oder $R^8$ durch Behandeln mit veresternden, solvolysierenden oder amidierenden Mitteln in einen anderen Rest $R^6$ und/oder $R^8$ umzuwandeln. So kann man eine Säure der Formel I ($R^6$ und/oder $R^8$ = OH) verestern, z. B. mit Hilfe eines Alkohols der Formel A-OH oder eines Diazoalkans, z. B. Diazomethan, oder man kann einen Ester der Formel I ($R^6$ und/oder $R^8$ = OA) zur entsprechenden Säure der Formel I ($R^6$ und/oder $R^8$ = OH) verseifen, z. B. mit wäßrig-dioxanischer Natronlauge bei Raumtemperatur. Ferner kann man z. B. einen der genannten Ester durch Behandeln mit Ammoniak oder mit einem Amin der Formel A-$NH_2$ oder $A_2NH$ in das entsprechende Amid der Formel I ($R^6$ und/oder $R^8$ = $NH_2$, NHA oder $NA_2$) überführen.

Es ist auch möglich, eine Thioethergruppe zu einer Sulfoxidgruppe, insbesondere eine Gruppe Y = Met zu einer Gruppe Y = Met(O) zu oxydieren, z. B. durch Einleiten von Luft in eine Lösung der Verbindung der Formel I (Y = Met) in Acetonitril/Wasser bei Temperaturen zwischen 0 und 30 °.

Umgekehrt kann man eine Sulfoxidgruppe (z. B. in I, Y = Met(O)) zu einer Thioethergruppe (z. B. in I, Y = Met) reduzieren, z. B.mit $NH_4J$ in wässeriger TFA bei Temperaturen zwischen -10 und 25°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein-oder mehrbasige Carbon-, Sulfon-oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-oder 3-Phenylpropionsäure, Citronensäure, Gluconsäure, Ascorbinsäure, Nicotinsäure, Isonicotinsäure, Methan-oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z. B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger-oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human-oder Veterinärmedizin eingesetzt werden. Als Trägersubstanzen kommen organische oder anorganische Stoffe in Frage, die sich für die enterale (z. B. orale oder rektale), parenterale oder lokale (z. B. topische) Applikation oder für eine Applikation in Form eines Inhalations-Sprays eignen und mit den neuen Verbindungen nicht reagieren, beispielsweise Wasser, niedere Alkohole, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose, Vaseline. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Zur topischen Anwendung eignen sich z. B. Lösungen, die in Form von Augentropfen verwendet werden können, ferner z. B. Suspensionen, Emulsionen, Cremes, Salben oder Komprimate. Für die Applikation als Inhalations-Spray können Sprays verwendet werden, die den Wirkstoff entweder gelöst oder suspendiert in einem Treibgasgemisch (z. B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabfolgt werden. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Konservierungs-, Stabilisierungs-und/oder Netzmittel, Emulgatoren, Salze zur Beeinflussung des osmotischen Druckes, Puffersubstanzen, Farb-und/oder Aromastoffe enthalten. Sie können, falls erwünscht, auch

einen oder mehrere weitere Wirkstoffe enthalten, z. B. ein oder mehrere Vitamine.

Die erfindungsgemäßen Substanzen werden in der Regel in Analogie zu anderen bekannten, im Handel befindlichen Peptiden, insbesondere aber in Analogie zu den in der US-A-4 472 305 beschriebenen Verbindungen verabreicht, vorzugsweise in Dosierungen zwischen etwa 0,05 und 500, insbesondere zwischen 0,5 und 100 mg pro Dosierungseinheit. Die tägliche Dosierung liegt vorzugsweise zwischen etwa 0,01 und 2 mg/kg Körpergewicht. Die spezielle Dosis für jeden bestimmten Patienten hängt jedoch von den ver schiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor-und nachstehend sind alle Temperaturen in °C angegeben. In den nachfolgenden Beispielen bedeutet "übliche Aufarbeitung": Man gibt, falls erforderlich, Wasser hinzu, neutralisiert, extrahiert mit Ether oder Dichlormethan, trennt ab, trocknet die organische Phase über Natriumsulfat, filtriert, dampft ein und reinigt durch Chromatographie an Kieselgel und/oder Kristallisation. RZ = Retentionszeit bei HPLC an RP 18 250-4-Säule, wenn nicht anders angegeben; Laufmittel: a = Wasser, b = 0,3 % TFA in Wasser, c = Acetonitril. $[\alpha] = [\alpha]_D^{20}$, c = 1 in Ethanol. Rf-Werte an Kieselgel Si 60 mit Elutionsmittel Dichlormethan/Methanol 9:1, wenn nicht anders angegeben.

Beispiel 1

Ein Gemisch von 907 mg L-Pyroglutamyl-L-(N-imi-2,4-dinitrophenyl-histidyl)-L-phenylalanyl-α-aminoisobutyryl-L-leucyl-methionin-amid [Glp-(imi-DNP-His)-Phe-Aib-Leu-Met-$NH_2$; erhältlich aus Glp-(imi-DNP-His)-OH und H-Phe-Aib-Leu-Met-$NH_2$ analog Beispiel 3, s. unten], 2 g 2-Mercaptoethanol, 20 ml DMF und 20 ml Wasser wird unter Rühren bei 20° mit wässeriger $Na_2CO_3$-Lösung auf pH 8 eingestellt und 2 Std. bei 20° gerührt. Nach üblicher Aufarbeitung erhält man L-Pyroglutamyl-L-histidyl-L-phenylalanyl-α-aminoisobutyryl-L-leucyl-methionin-amid (Glp-His-Phe-Aib-Leu-Met-$NH_2$).

Beispiel 2

Man löst 1 g Glp-Phe-(imi-BOM-His)-Aib-Leu-Nle-$NH_2$ [erhältlich aus Glp-Phe-(imi-BOM-His)-OH und H-Aib-Leu-Nle-$NH_2$ analog Beispiel 3, s. unten] in 25 ml Methanol, hydriert 3 Std. an 0,5 %ig. Pd-C bei 20° und 1 bar, filtriert, dampft ein und erhält nach üblicher Aufarbeitung Glp-Phe-His-Aib-Leu-Nle-$NH_2$.

Beispiel 3

Eine Lösung von H-D-Phe-Aib-Leu-Met-$NH_2$ HCl (s. Beispiel 12) in 60 ml Dichlormethan wird unter Kühlung mit 2,02 g N-Methylmorpholin versetzt. Unter Rühren gibt man 2,86 g Glp-Phe-OH, 1,35 g HOBt und eine Lösung von 1,92 g EDCI in 50 ml Dichlormethan hinzu, rührt 14 Std. bei 4°, dampft ein, arbeitet wie üblich auf und erhält Glp-Phe-D-Phe-Aib-Leu-Met-$NH_2$, F. 116°.

Beispiel 4

Analog Beispiel 3 erhält man aus 3,51 g H-Aib-Leu-Met-$NH_2$ . HCl mit 2,02 g N-Methylmorpholin, 4,23 g Glp-Phe-D-Phe-OH [F. ab 220° (Zers.); erhältlich durch Kondensation von BOC-Phe-OH mit H-D-Phe-OMe zu BOC-Phe-D-Phe-OMe (F. 125-128°), Abspaltung der BOC-Gruppe und Kondensation mit Glp-OH zu Glp-Phe-D-Phe-OMe und Verseifung mit NaOH in Dioxan], 1,35 g HOBt und 1,92 g EDCI in 110 ml Dichlormethan das Glp-Phe-D-Phe-Aib-Leu-Met-$NH_2$, F. 116°.

Analog erhält man:

Glp-Phe-Phe-Aib-Leu-Met-$NH_2$, F. 170°
CBZ-Asp(OBut)-Phe-Phe-Val-Aib-Leu-Met-$NH_2$
BOC-Lys(CBZ)-D-Trp-D-Val-Aib-Leu-Met-$NH_2$, RZ 20,65 (b/c 1:1)
BOC-Lys(CBZ)-D-Trp-D-Phe-Aib-Leu-Met-$NH_2$, RZ 11,51 (b/c 6:4)
BOC-D-Trp-Phe-Aib-Leu-Met-$NH_2$, RZ 15,11 (b/c 1:1)

BOC-D-Trp-D-Phe-Aib-Leu-Met-$NH_2$, RZ 16,19 (b/c 1:1)
Glp-Phe-Phe-Deg-Leu-Met-$NH_2$
Glp-Phe-D-Phe-Deg-Leu-Met-$NH_2$
Glp-Phe-Phe-Dpg-Leu-Met-$NH_2$
Glp-Phe-Phe-Dpg-Leu-Met-OMe
Glp-Phe-D-Phe-Dpg-Leu-Met-$NH_2$
Glp-Phe-D-Phe-Dpg-Leu-Met-OMe
BOC-Lys(CBZ)-D-Trp-D-Phe-Dpg-Leu-Met-$NH_2$.

Beispiel 5

Analog Beispiel 3 erhält man aus Glp-Phe-D-Phe-OH und 2S-(2-Oxo-piperazino)-4-methyl-valeryl-Met-$NH_2$ das 2S-[2-Oxo-4-(Glp-Phe-D-Phe)-piperazino]-4-methyl-valeryl-Met-$NH_2$, RZ 5,9 (b/c 6:4).

Analog erhält man mit den entsprechenden 1-substituierten 2-Oxopiperazinen:
2S-[2-Oxo-4-(Glp-Phe-D-Phe)-piperazino]-4-methyl-valeryl-Met-OMe, RZ 13, 1 (b/c 6:4)
2S-[2-Oxo-4-(Glp-Phe-Phe)-piperazino]-4-methyl-valeryl-Met-$NH_2$, RZ 4,7 (b/c 6:4)
2S-[2-Oxo-4-(Glp-Phe-Phe)-piperazino]-4-methyl-valeryl-Met-OMe, RZ 11,9 (b/c 6:4)
2S-[2-Oxo-4-(CBZ-Asp(OBut)-Phe-Phe-Val)-piperazino]-4-methyl-valeryl-Met-$NH_2$, RZ 9,0 (b/c 1:1)
2S-[2-Oxo-4-(CBZ-Asp(OBut)-Phe-Phe-Val)-piperazino]-4-methyl-valeryl-Met-OMe
2S-[2-Oxo-4-(BOC-Lys(CBZ)-D-Trp-D-Phe)-piperazino]-4-methyl-valeryl-Met-$NH_2$, RZ 6,6 (b/c 4:6)
2S-[2-Oxo-4-(CBZ-Asp(OBut)-Phe-Phe-Val)-piperazino]-4-methyl-valeryl-Met-OMe, RZ 12,1 (b/c 4:6)
2S-[2-Oxo-4-(BOC-Phe-Phe-Val)-piperazino]-4-methyl-valeryl-Met-$NH_2$
2S-[2-Oxo-4-(BOC-Phe-Phe-Val)-piperazino]-4-methyl-valeryl-Met-OMe.

Beispiel 6

Analog Beispiel 3 erhält man aus Glp-Phe-Phe-OH und H-Cle-Leu-Met-OMe das Glp-Phe-Phe-Cle-Leu-Met-OMe, F. 106°.

Analog erhält man

Glp-Phe-Phe-Cle-Leu-Met-$NH_2$, F. 186°
Glp-Phe-D-Phe-Cle-Leu-Met-OMe, F. 88°
Glp-Phe-D-Phe-Cle-Leu-Met-$NH_2$, RZ 10,5 (b/c 6:4)
BOC-Lys(CBZ)-D-Trp-D-Phe-Cle-Leu-Met-$NH_2$, RZ 21,3 (b/c 1:1).

Beispiel 7

Analog Beispiel 3 erhält man aus BOC-Lys(CBZ)-D-Trp-D-Phe-OH [F. 81-84°; erhältlich durch Kondensation von BOC-D-Trp-OH mit H-D-Phe-OH zu BOC-D-Trp-D-Phe-OH (F. 103-105°), Abspaltung der BOC-Gruppe mit HCl in Dioxan zu H-D-Trp-D-Phe-OH und Kondensation mit BOC-Lys(CBZ)-OH] und 2S-(2-Oxo-3S-amino-pyrrolidino)-4-methyl-valeryl-Met-$NH_2$ (s. Beispiel 12) das 2S-[2-Oxo-3S-(BOC-Lys(CBZ)-D-Trp-D-Phe-amino)-pyrrolidino]-4-methyl-valeryl-Met-$NH_2$, RZ 8,94 (a/c 4:6); Rf 0,48.

Analog erhält man

2S-[2-Oxo-3R-(BOC-Lys(CBZ)-D-Trp-D-Phe-amino)-pyrrolidino]-4-methyl-valeryl-Met-$NH_2$, RZ 7,09 (b/c 4:6)
2R-[2-Oxo-3S-(BOC-Lys(CBZ)-D-Trp-D-Phe-amino)-pyrrolidino]-4-methyl-valeryl-Met-$NH_2$, RZ 8,49 (a/c 4:6)
2R-[2-Oxo-3R-(BOC-Lys(CBZ)-D-Trp-D-Phe-amino)-pyrrolidino]-4-methyl-valeryl-Met-$NH_2$, RZ 10,25 (a/c 1:1)
2S-[2-Oxo-3S-(BOC-Lys(CBZ)-D-Trp-D-Phe-amino)-pyrrolidino]-4-methyl-valeryl-Met-OMe, RZ 15,39 (b/c 4:6)
2S-[2-Oxo-3R-(BOC-Lys(CBZ)-D-Trp-D-Phe-amino)-pyrrolidino]-4-methyl-valeryl-Met-OMe, RZ 5,49 (b/c 33:65)
2R-[2-Oxo-3S-(BOC-Lys(CBZ)-D-Trp-D-Phe-amino)-pyrrolidinol-4-methyl-valeryl-Met-OMe, RZ 14,72 (b/c 4:6)
2R-[2-Oxo-3R-(BOC-Lys(CBZ)-D-Trp-D-Phe-amino)-pyrrolidino]-4-methyl-valeryl-Met-OMe, RZ 16,16 (b/c

4:6)
2S-[2-Oxo-3S-(BOC-Lys(CBZ)-Phe-Phe-amino)-pyrrolidino]-4-methyl-valeryl-Phe-$NH_2$, F. 115-123°
2S-[2-Oxo-3S-(BOC-D-Trp-Phe-amino)-pyrrolidino]-4-methyl-valeryl-Met-$NH_2$, F. 125-132°
2S-[2-Oxo-3S-(BOC-D-Trp-Phe-amino)-pyrrolidino]-4-methyl-valeryl-Phe-$NH_2$, F. 135-145°
2S-[2-Oxo-3R-(BOC-D-NαMeTrp-Phe-amino)-pyrrolidino]-4-methyl-valeryl-Nle-$NH_2$, F. 128-133°
2S-[2-Oxo-3S-(BOC-D-Pro-D-Trp-Phe-amino)-pyrrolidino]-4-methyl-valeryl-Met-$NH_2$, Rf 0,51
2S-[2-Oxo-3S-(BOC-Lys(CBZ)-D-Trp-Phe-amino)-pyrrolidino]-4-methyl-valeryl-Met-$NH_2$, Rf 0,53
2S-[2-Oxo-3S-(BOC-Lys(CBZ)-D-Trp-D-Phe-amino)-pyrrolidino]-4-methyl-valeryl-Ile-$NH_2$
2S-[2-Oxo-3S-(BOC-Lys(CBZ)-D-Trp-D-Phe-amino)-pyrrolidino]-4-methyl-valeryl-Leu-$NH_2$
2S-[2-Oxo-3S-(BOC-Lys(CBZ)-D-Trp-D-Phe-amino)-pyrrolidino]-4-methyl-valeryl-Nle-$NH_2$
2S-[2-Oxo-3S-(BOC-Lys(CBZ)-D-Trp-D-Phe-amino)-pyrrolidino]-4-methyl-valeryl-Phe-$NH_2$
2S-[2-Oxo-3S-(BOC-Lys(CBZ)-Phe-Phe-amino)-pyrrolidino]-4-methyl-valeryl-Met-$NH_2$, Rf 0,43
2S-[2-Oxo-3R-(BOC-D-Pro-D-Trp-Phe-amino)-pyrrolidino]-4-methyl-valeryl-Nle-$NH_2$, Rf 0,36
2S-[2-Oxo-3R-(BOC-D-Pro-D-Trp-D-Phe-amino)-pyrrolidino]-4-methyl-valeryl-Nle-$NH_2$, Rf 0,32
2S-[2-Oxo-3S-(BOC-D-Pro-D-Trp-D-Phe-amino)-pyrrolidino]-4-methyl-valeryl-Phe-$NH_2$, Rf 0,36
2S-[2-Oxo-3S-(BOC-D-Pro-D-Trp-Phe-amino)-pyrrolidino]-4-methyl-valeryl-Phe-$NH_2$, Rf 0,42
2S-[2-Oxo-3S-(BOC-Lys(CBZ)-D-Trp-D-Phe-amino)-pyrrolidino]-4-methyl-valeryl-Met(O)-$NH_2$, Rf 0,68 ($CHCl_3$/$CH_3OH$/$CH_3COOH$ 85:10:5)
2S-[2-Oxo-3S-(BOC-Lys(CBZ)-D-Phe-D-Phe-amino)-pyrrolidino]-4-methyl-valeryl-Met-$NH_2$
2S-[2-Oxo-3S-(BOC-Lys(CBZ)-D-αNal-D-Phe-amino)-pyrrolidino]-4-methyl-valeryl-Met-$NH_2$
2S-[2-Oxo-3S-(BOC-Lys(CBZ)-D-N-Me-Trp-D-Phe-amino)-pyrrolidino]-4-methyl-valeryl-Met-$NH_2$
2S-[2-Oxo-3S-(BOC-Lys(CBZ)-D-CαMeTrp-D-Phe-amino)-pyrrolidino]-4-methyl-valeryl-Met-$NH_2$
2S-[2-Oxo-3S-(BOC-Lys(CBZ)-D-Tyr-D-Phe-amino)-pyrrolidino]-4-methyl-valeryl-Met-$NH_2$
2S-[2-Oxo-3S-(BOC-Asn-D-Trp-D-Phe-amino)-pyrrolidino]-4-methyl-valeryl-Met-$NH_2$
2S-[2-Oxo-3S-(BOC-Gln-D-Trp-D-Phe-amino)-pyrrolidino]-4-methyl-valeryl-Met-$NH_2$
2S-[2-Oxo-3S-(BOC-Orn(CBZ)-D-Trp-D-Phe-amino)-pyrrolidino]-4-methyl-valeryl-Met-$NH_2$
2S-[2-Oxo-3S-(BOC-Pro-D-Trp-D-Phe-amino)-pyrrolidino]-4-methyl-valeryl-Met-$NH_2$
2S-[2-Oxo-3S-(BOC-Lys(CBZ)-D-Trp-Phe-amino)-pyrrolidino]-4-methyl-valeryl-Met-$NH_2$
2S-[2-Oxo-3S-(BOC-Lys(CBZ)-D-Trp-Ile-amino)-pyrrolidino]-4-methyl-valeryl-Met-$NH_2$
2S-[2-Oxo-3S-(BOC-Lys(CBZ)-D-Trp-D-Ile-amino)-pyrrolidino]-4-methyl-valeryl-Met-$NH_2$
2S-[2-Oxo-3S-(BOC-Lys(CBZ)-D-Trp-Leu-amino)-pyrrolidino]-4-methyl-valeryl-Met-$NH_2$
2S-[2-Oxo-3S-(BOC-Lys(CBZ)-D-Trp-N-Me-Phe-amino)-pyrrolidino]-4-methyl-valeryl-Met-$NH_2$
2S-[2-Oxo-3S-(BOC-Lys(CBZ)-D-Trp-Trp-amino)-pyrrolidino]-4-methyl-valeryl-Met-$NH_2$
2S-[2-Oxo-3S-(BOC-Lys(CBZ)-D-Trp-Val-amino)-pyrrolidino]-4-methyl-valeryl-Met-$NH_2$
2S-[2-Oxo-3S-(BOC-Lys(CBZ)-D-Trp-D-Val-amino)-pyrrolidino]-4-methyl-valeryl-Met-$NH_2$
2S-[2-Oxo-3S-(N-(Glp-Phe-D-Phe)-N-methyl-amino)-pyrrolidino]-4-methyl-valeryl-Met-$NH_2$, RZ 6,67 Min. (b/c 4:6)
2S-[2-Oxo-3S-(N-(Glp-Phe-Phe)-N-methyl-amino)-pyrrolidino]-4-methyl-valeryl-Met-$NH_2$, RZ 10,06 Min. (a/c 6:4).

Beispiel 8

Analog Beispiel 3 erhält man aus BOC-Aib-OH und H-Leu-Met-$NH_2$ [erhältlich durch Kondensation von BOC-Leu-OH mit H-Met-$NH_2$ zu BOC-Leu-Met-NH2 (F 156°) und Abspaltung der BOC-Gruppe] das BOC-Aib-Leu-Met-$NH_2$, F. 82-90°.

Analog erhält man

BOC-Aib-Leu-Met-OMe
BOC-Deg-Leu-Met-$NH_2$
BOC-Dpg-Leu-Met-$NH_2$
BOC-D-Phe-Aib-Leu-Met-OMe
BOC-D-Phe-Aib-Leu-Met-$NH_2$
BOC-Cle-Leu-Met-OMe
BOC-Cle-Leu-Met-$NH_2$

Beispiel 9

Analog Beispiel 3 erhält man aus Glp-Phe-D-Phe-Aib-Leu-OH und H-Met-$NH_2$ das Glp-Phe-D-Phe-Aib-Leu-Met-$NH_2$, F. 116°.

Analog erhält man mit 2S-(2-Oxo-4-BOC-piperazino)-4-methyl-valeriansäure das 2S-(2-Oxo-4-BOC-piperazino)-4-methyl-valeryl-Met-$NH_2$, F. 61°.

Analog erhält man mit 2R-bzw. 2S-(2-Oxo-3R-bzw. 3S-BOC-amino-pyrrolidino)-4-methylvaleriansäure:

2R-(2-Oxo-3R-BOC-amino-pyrrolidino)-4-methyl-valeryl-Met-$NH_2$, [α] +66,3°
2R-(2-Oxo-3S-BOC-amino-pyrrolidino)-4-methyl-valeryl-Met-$NH_2$, [α] +17,1°
2S-(2-Oxo-3R-BOC-amino-pyrrolidino)-4-methyl-valeryl-Met-$NH_2$, [α] -41,5°
2S-(2-Oxo-3S-BOC-amino-pyrrolidino)-4-methyl-valeryl-Met-$NH_2$, F. 163-165°; [α] -76,6°.

Analog erhält man mit 2S-[2-Oxo-4-(CBZ-Phe-Phe-Val)-piperazino]-4-methylvaleriansäure das 2S-[2-Oxo-4-(CBZ-Phe-Phe-Val)-piperazino]-4-methyl-valeryl-Met-$NH_2$, RZ 15,17 (b/c 55:45).

Beispiel 10

Analog Beispiel 3 erhält man aus Glp-Phe-D-Phe-Aib-Leu-Met-OH und Diethylamin das Glp-Phe-D-Phe-Aib-Leu-Met-$N(C_2H_5)_2$.

Analog erhält man mit den entsprechenden Aminen:
Glp-Phe-D-Phe-Aib-Leu-Met-$NHCH_3$
Glp-Phe-D-Phe-Aib-Leu-Met-$N(CH_3)_2$
Glp-Phe-D-Phe-Aib-Leu-Met-$NHC_2H_5$
Glp-Phe-D-Phe-Aib-Leu-Met-$N(C_4H_9)_2$
Glp-Phe-D-Phe-Aib-Leu-Met-$N(C_8H_{17})_2$.

Beispiel 11

Eine Lösung von 0,1 g 2S-[2-Oxo-3S-(BOC-Lys(CBZ)-D-Trp-D-Phe-amino)-pyrrolidino]-4-methyl-valeryl-Met-$NH_2$ in 10 ml einer 40 %igen Lösung von TFA in Dichlormethan wird 16 Std. bei 10° stehengelassen. Nach dem Eindampfen und chromatographischer Aufreinigung erhält man 2S-[2-Oxo-3S-(H-Lys(CBZ)-D-Trp-D-Phe-amino)-pyrrolidino]-4-methyl-valeryl-Met-$NH_2$, Tfa, F. 107°; RZ 4,4 (b/c 4:6).

Analog erhält man durch Spaltung der entsprechenden BOC-Derivate:

2S-[2-Oxo-3R-(H-Lys(CBZ)-D-Trp-D-Phe-amino)-pyrrolidino]-4-methyl-valeryl-Met-$NH_2$, RZ 5,5 (b/c 1:1)
2R-[2-Oxo-3S-(H-Lys(CBZ)-D-Trp-D-Phe-amino)-pyrrolidino]-4-methyl-valeryl-Met-$NH_2$
2R-[2-Oxo-3R-(H-Lys(CBZ)-D-Trp-D-Phe-amino)-pyrrolidino]-4-methyl-valeryl-Met-$NH_2$
2S-[2-Oxo-3S-(H-Lys(CBZ)-D-Trp-D-Phe-amino)-pyrrolidino]-4-methyl-valeryl-Met-OMe, RZ 6,2 (b/c 4:6)
2S-[2-Oxo-3R-(H-Lys(CBZ)-D-Trp-D-Phe-amino)-pyrrolidino]-4-methyl-valeryl-Met-OMe, RZ 5,6 (b/c 4:6)
2R-[2-Oxo-3S-(H-Lys(CBZ)-D-Tp-D-Phe-amino)-pyrrolidino]-4-methyl-valeryl-Met-OMe, RZ 6,6 (b/c 4:6)
2R-[2-Oxo-3R-(H-Lys(CBZ)-D-Trp-D-Phe-amino)-pyrrolidino]-4-methyl-valeryl-Met-OMe, RZ 6,6 (b/c 4:6)
2S-[2-Oxo-3S-(H-Lys(CBZ)-D-Trp-Phe-amino)-pyrrolidino]-4-methyl-valeryl-Phe-$NH_2$, Tfa, F. 130°
2S-[2-Oxo-3S-(H-Lys(CBZ)-Phe-Phe-amino)-pyrrolidino]-4-methyl-valeryl-Phe-$NH_2$, Tfa, F. 118°
2S-[2-Oxo-3S-(H-D-Trp-Phe-amino)-pyrrolidino]-4-methyl-valeryl-Met-$NH_2$, Tfa, F. 141°
2S-[2-Oxo-3S-(H-D-Trp-Phe-amino)-pyrrolidino]-4-methyl-valeryl-Phe-$NH_2$, Tfa, F. 152°
2S-[2-Oxo-3R-(H-D-NαMeTrp-Phe-amino)-pyrrolidino]-4-methyl-valeryl-Nle-$NH_2$, Tfa, F. 143°
2S-[2-Oxo-3S-(H-D-Pro-D-Trp-Phe-amino)-pyrrolidino]-4-methyl-valeryl-Met-$NH_2$, Tfa, F. 129°
2S-[2-Oxo-3S-(H-Lys(CBZ)-D-Trp-Phe-amino)-pyrrolidino]-4-methyl-valeryl-Met-$NH_2$, Tfa, F. 133°
2S-[2-Oxo-3S-(H-Lys(CBZ)-D-Trp-D-Phe-amino)-pyrrolidino]-4-methyl-valeryl-Ile-$NH_2$
2S-[2-Oxo-3S-(H-Lys(CBZ)-D-Trp-D-Phe-amino)-pyrrolidino]-4-methyl-valeryl-Leu-$NH_2$
2S-[2-Oxo-3S-(H-Lys(CBZ)-D-Trp-D-Phe-amino)-pyrrolidino]-4-methyl-valeryl-Nle-$NH_2$
2S-[2-Oxo-3S-(H-Lys(CBZ)-D-Trp-D-Phe-amino)-pyrrolidino]-4-methyl-valeryl-Phe-$NH_2$
2S-[2-Oxo-3S-(H-Lys(CBZ)-Phe-Phe-amino)-pyrrolidino]-4-methyl-valeryl-Met-$NH_2$, Tfa, F. 118°
2S-[2-Oxo-3R-(H-D-Pro-D-Trp-Phe-amino)-pyrrolidino]-4-methyl-valeryl-Nle-$NH_2$, Tfa, F. 146°
2S-[2-Oxo-3R-(H-D-Pro-D-Trp-D-Phe-amino)-pyrrolidino]-4-methyl-valeryl-Nle-$NH_2$, Tfa, F. 164-170°
2S-[2-Oxo-3S-(H-D-Pro-D-Trp-D-Phe-amino)-pyrrolidino]-4-methyl-valeryl-Phe-$NH_2$, Tfa, F. 144°

2S-[2-Oxo-3S-(H-D-Pro-D-Trp-Phe-amino)-pyrrolidino]-4-methyl-valeryl-Phe-$NH_2$, Tfa, F. 156°
2S-[2-Oxo-3S-(H-Lys(CBZ)-D-Trp-D-Phe-amino)-pyrrolidino]-4-methyl-valeryl-Met(O)-$NH_2$, Tfa, F. 123°
2S-[2-Oxo-3S-(H-Lys(CBZ)-D-Phe-D-Phe-amino)-pyrrolidino]-4-methyl-valeryl-Met-$NH_2$
2S-[2-Oxo-3S-(H-Lys(CBZ)-D-αNal-D-Phe-amino)-pyrrolidino]-4-methyl-valeryl-Met-$NH_2$
2S-[2-Oxo-3S-(H-Lys(CBZ)-D-N-Me-Trp-D-Phe-amino)-pyrrolidino]-4-methyl-valeryl-Met-$NH_2$
2S-[2-Oxo-3S-(H-Lys(CBZ)-D-CαMeTrp-D-Phe-amino)-pyrrolidino]-4-methyl-valeryl-Met-$NH_2$
2S-[2-Oxo-3S-(H-Lys(CBZ)-D-Tyr-D-Phe-amino)-pyrrolidino]-4-methyl-valeryl-Met-$NH_2$
2S-[2-Oxo-3S-(H-Asn-D-Trp-D-Phe-D-Phe-amino)-pyrrolidino]-4-methyl-valeryl-Met-$NH_2$
2S-[2-Oxo-3S-(H-Gln-D-Trp-D-Phe-D-Phe-amino)-pyrrolidino]-4-methyl-valeryl-Met-$NH_2$
2S-[2-Oxo-3S-(H-Orn(CBZ)-D-Trp-D-Phe-D-Phe-amino)-pyrrolidino]-4-methyl-valeryl-Met-$NH_2$
2S-[2-Oxo-3S-(H-Pro-D-Trp-D-Phe-amino)-pyrrolidino]-4-methyl-valeryl-Met-$NH_2$
2S-[2-Oxo-3S-(H-Lys(CBZ)-D-Trp-Phe-amino)-pyrrolidino]-4-methyl-valeryl-Met-$NH_2$
2S-[2-Oxo-3S-(H-Lys(CBZ)-D-Trp-Ile-amino)-pyrrolidino]-4-methyl-valeryl-Met-$NH_2$
2S-[2-Oxo-3S-(H-Lys(CBZ)-D-Trp-D-Ile-amino)-pyrrolidino]-4-methyl-valeryl-Met-$NH_2$
2S-[2-Oxo-3S-(H-Lys(CBZ)-D-Trp-Leu-amino)-pyrrolidino]-4-methyl-valeryl-Met-$NH_2$
2S-[2-Oxo-3S-(H-Lys(CBZ)-D-Trp-N-Me-Phe-amino)-pyrrolidino]-4-methyl-valeryl-Met-$NH_2$
2S-[2-Oxo-3S-(H-Lys(CBZ)-D-Trp-Trp-amino)-pyrrolidino]-4-methyl-valeryl-Met-$NH_2$
2S-[2-Oxo-3S-(H-Lys(CBZ)-D-Trp-Val-amino)-pyrrolidino]-4-methyl-valeryl-Met-$NH_2$
2S-[2-Oxo-3S-(H-Lys(CBZ)-D-Trp-D-Val-amino)-pyrrolidino]-4-methyl-valeryl-Met-$NH_2$
2S-[2-Oxo-4-(H-Lys(CBZ)-D-Trp-D-Phe)-piperazino]-4-methyl-valeryl-Met-$NH_2$, RZ 8,5 (b/c 55:45)
2S-[2-Oxo-4-(H-Lys(CBZ)-D-Trp-D-Phe)-piperazino]-4-methyl-valeryl-Met-OMe, RZ 10,3 (b/c 1:1)
2S-[2-Oxo-4-(H-Phe-Phe-Val)-piperazino]-4-methyl-valeryl-Met-$NH_2$, RZ 4,48 (b/c 6:4)
H-Lys(CBZ)-D-Trp-D-Val-Aib-Leu-Met-$NH_2$, RZ 11,51 (b/c 6:4)
H-Lys(CBZ)-D-Trp-D-Phe-Aib-Leu-Met-$NH_2$
H-Lys(CBZ)-D-Trp-D-Phe-Dpg-Leu-Met-$NH_2$
H-Lys(CBZ)-D-Trp-D-Phe-Cle-Leu-Met-$NH_2$

Beispiel 12

Eine Lösung von 1 g 2S-[2-Oxo-4-(CBZ-Asp(OBut)-Phe-Phe-Val)-piperazino]-4-methyl-valeryl-Met-$NH_2$ in 15 ml 4 n HCl in Dioxan wird 2 Std. bei 20° gerührt. Man dampft ein, arbeitet wie üblich auf und erhält 2S-[2-Oxo-4-(CBZ-Asp-Phe-Phe-Val)-piperazino]-4-methyl-valeryl-Met-$NH_2$, RZ 4,1 Min. (b/c 4:6).

Analog erhält man aus dem entsprechenden Asp(OBut)-Derivat:

CBZ-Asp-Phe-Phe-Val-Aib-Leu-Met-$NH_2$.

Analog erhält man durch Spaltung der entsprechenden BOC-Derivate:

H-Aib-Leu-Met-OMe
H-Aib-Leu-Met-$NH_2$
H-Deg-Leu-Met-$NH_2$
H-Dpg-Leu-Met-$NH_2$
H-D-Phe-Aib-Leu-Met-OMe
H-D-Phe-Aib-Leu-Met-$NH_2$
H-Cle-Leu-Met-OMe
H-Cle-Leu-Met-$NH_2$
2S-(2-Oxo-piperazino)-4-methyl-valeryl-Met-$NH_2$, F. 104°
2R-(2-Oxo-2R-amino-pyrrolidino)-4-methyl-valeryl-Met-$NH_2$
2R-(2-Oxo-3S-amino-pyrrolidino)-4-methyl-valeryl-Met-$NH_2$
2S-(2-Oxo-3R-amino-pyrrolidino)-4-methyl-valeryl-Met-$NH_2$
2S-(2-Oxo-3S-amino-pyrrolidino)-4-methyl-valeryl-Met-$NH_2$.

Beispiel 13

Zu einer Lösung von 50 mg 2S-[2-Oxo-4-(BOC-Lys(CBZ)-D-Trp-D-Phe)-piperazino]-4-methyl-valeryl-Met-$NH_2$ in 5 ml Methanol und 5 ml DMF gibt man unter $N_2$ 5 mg 10 %ig Pd-C und dann 10 mg Ammoniumformiat, verschließt den Kolben, rührt 5 Tage bei 20°, filtriert, konzentriert die Lösung und erhält 2S-[2-Oxo-4-(BOC-Lys-D-Trp-D-Phe)-piperazino]-4-methyl-valeryl-Met-$NH_2$; RZ 9,3 Min. (b/c 6:4).

Analog erhält man aus 2S-[2-Oxo-4-(CBZ-Asp-Phe-Phe-Val)-piperazino]-4-methyl-valeryl-Met-$NH_2$ das 2S-[2-Oxo-4-(H-Asp-Phe-Phe-Val)-piperazino]-4-methyl-valeryl-Met-$NH_2$; RZ 4,3 Min. (b/c: 6:4).

Beispiel 14

Zu einer Lösung von 55 mg 2S-[2-Oxo-4-H-Phe-Phe-Val)-piperazino]-4-methyl-valeryl-Met-$NH_2$-hydrochlorid in einem Gemisch von 10 ml Dichlormethan und 1 ml DMF gibt man 6,8 mg Bernsteinsäureanhydrid und 6,8 mg Triethylamin, rührt 24 Std., konzentriert, chromatographiert den Rückstand und erhält 2S-[2-Oxo-4-(SuccPhe-Phe-Val)-piperazino]-4-methyl-valeryl-Met-$NH_2$, RZ 8,3 (b/c 6:4).

Beispiel 15

Eine Lösung von 100 mg 2S-[2-Oxo-4-(BOC-Lys(CBZ)-D-Trp-D-Phe)-piperazino]-4-methyl-valeryl-Met-OH in 60 ml Dioxan wird mit einer Lösung von $CH_2N_2$ in Dioxan bis zur bleibenden Gelbfärbung versetzt. Man dampft ein, arbeitet wie üblich auf und erhält 2S-[2-Oxo-4-(BOC-Lys(CBZ)-D-Trp-D-Phe)-piperazino]-4-methyl-valeryl-Met-OMe.

Beispiel 16

Eine Lösung von 50 mg 2S-[2-Oxo-4-(BOC-Lys(CBZ)-D-Trp-D-Phe)-piperazino]-4-methyl-valeryl-Met-OMe in einem Gemisch von 5 ml 0,1 n wässeriger Natronlauge und 5 ml Dioxan wird 24 Std. bei 20 ° stehengelassen. Man gibt 1 n Salzsäure bis pH 1 hinzu, dampft ein, chromatographiert den Rückstand an RP 18 (0,3 %ig. TFA in Wasser/$CH_3CN$) und erhält 2S-[2-Oxo-4-(BOC-Lys(CBZ)-D-Trp-D-Phe)-piperazino]-4-methyl-valeryl-Met-OH.

Beispiel 17

Eine Lösung von 50 mg 2S-[2-Oxo-4-(BCO-Lys(CBZ)-D-Trp-D-Phe)-piperazino]-4-methyl-valeryl-Met-OMe in 5 ml Methanol und 5 ml 25%iger wässeriger $NH_3$-Lösung wird mit $NH_4Cl$ gesättigt. Man leitet 24 Std. $NH_3$ ein, konzentriert die Lösung und arbeitet wie üblich auf. Nach Chromatographie (Dichlormethan/Methanol 98:2) erhält man 2S-[2-Oxo-4-(BOC-Lys(CBZ)-D-Trp-D-Phe)-piperazino]-4-methyl-valeryl-Met-$NH_2$; RZ 6,6 Min. (b/c 4:6).

Beispiel 18

Man leitet Luft durch eine Lösung von 1 g 2S-[2-Oxo-3S-(H-Lys(CBZ)-D-Trp-D-Phe-amino)-pyrrolidino]-4-methyl-butyryl-Met-$NH_2$ in 50 ml Acetonitril und 50 ml Wasser bis zur vollständigen Umsetzung (chromatographische Kontrolle). Nach üblicher Aufarbeitung erhält man das entsprechende Sulfoxid, 2S-[2-Oxo-3S-(H-Lys(CBZ)-D-Trp-D-Phe-amino)-pyrrolidino]-4-methyl-butyryl-Met(O)-$NH_2$, Tfa, F. 123°.

Analog erhält man

Glp-Phe-D-Phe-Aib-Leu-Met(O)-$NH_2$
2S-[2-Oxo-4-(CBZ-Asp-Phe-Phe-Val)-piperazino]-4-methyl-butyryl-Met(O)-$NH_2$
2S-[2-Oxo-4-(H-Asp-Phe-Phe-Val)-piperazino]-4-methyl-butyryl-Met(O)-$NH_2$
Glp-Phe-Phe-Cle-Leu-Met(O)-OMe
Glp-Phe-D-Phe-Cle-Leu-Met(O)-OMe.

Beispiel 19

Eine Lösung von 1 g 2S-[2-Oxo-4-(H-Lys(CBZ)-D-Trp-D-Phe-amino)-pyrrolidino]-4-methyl-butyryl-Met-(O)-$NH_2$ in 50 ml TFA wird bei 0° mit 20 ml 2 m $NH_4J$-Lösung versetzt. Nach 1 Std. Rühren bei 0° reduziert man das entstandene Jod durch Zugabe von Thioglykolsäure, arbeitet wie üblich auf und erhält 2S-[2-Oxo-4-(H-Lys(CBZ)-D-Trp-D-Phe-amino)-pyrrolidino]-4-methyl-butyryl-Met-$NH_2$.

Beispiel 20

Analog Beispiel 5 erhält man:

2S-[2-Oxo-4-(CBZ-Phe-Val)-piperazino]-4-methyl-valeryl-Met-$NH_2$, RZ 19,77 (a/c 6:4)
2S-[2-Oxo-4-(BOC-D-Trp-Phe)-piperazino]-4-methyl-valeryl-Phe-$NH_2$, RZ 9,39 (a/c 1:1)
2S-[2-Oxo-4-(D-Pro-D-Trp-Phe)-piperazino]-4-methyl-valeryl-Phe-$NH_2$, RZ 10,83 (b/c 65:35)
2S-[2-Oxo-4-(Lys(CBZ)-D-Trp-Phe)-piperazino]-4-methyl-valeryl-Phe-$NH_2$
2S-[2-Oxo-4-(D-Trp-Phe)-piperazino]-4-methyl-valeryl-Phe-$NH_2$
2S-[2-Oxo-4-(D-Trp-Phe)-piperazino]-4-methyl-valeryl-Met-$NH_2$, RZ 7,7 (b/c 65:35)
2S-[2-Oxo-4-(Succ-Phe-Val)-piperazino]-4-methyl-valeryl-Met-$NH_2$, RZ 9,41 (b/c 7:3)
2S-[2-Oxo-4-(phenylacetyl-Phe-Val)-piperazino]-4-methyl-valeryl-Met-$NH_2$, RZ 23,57 (b/c 65:35)
2S-[2-Oxo-4-(benzoyl-Phe-Val)-piperazino]-4-methyl-valeryl-Met-$NH_2$, RZ 7,6 (b/c 7:3)
2S-[2-Oxo-4-(p-aminophenylacetyl-Phe-Val)-piperazino]-4-methyl-valeryl-Met-$NH_2$, RZ 10,69 (b/c 75:25)
2S-[2-Oxo-4-(Glp-Phe-Ile)-piperazino]-4-methyl-valeryl-Met-$NH_2$, RZ 12,83 (b/c 7:3)
2S-[2-Oxo-4-(BOC-Pro-N-Me-Phe-N-Me-Phe)-piperazino]-4-methyl-valeryl-Met-$NH_2$, RZ 16,99 (b/c 55:45)
2S-[2-Oxo-4-(acetyl-Phe-Val)-piperazino]-4-methyl-valeryl-Met-$NH_2$, RZ 6,64 (b/c 65:35)
2S-[2-Oxo-4-(CBZ-Phe-Phe)-piperazino]-4-methyl-valerylMet-$NH_2$, RZ 18,75 (a/c 6:4)
2S-[2-Oxo-4-(Pro-N-Me-Phe-N-Me-Phe)-piperazino]-4-methyl-valeryl-Met-$NH_2$.

Die nachstehenden Beispiele betreffen pharmazeutische Zubereitungen.

Beispiel A: Injektionsgläser

Eine Lösung von 100 g 2S-[2-Oxo-4-(H-Asp-Phe-Phe-Val)-piperazino]-4-methyl-valeryl-Met-$NH_2$ und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

Beispiel B: Suppositorien

Man schmilzt ein Gemisch von 20 g Glp-Phe-D-Phe-Aib-Leu-Met-$NH_2$ mit 100 g Sojalecithin und 1400 g Kakaobutter, gießt in Formen und läßt erkalten. Jedes Suppositorium enthält 20 mg Wirkstoff.

Beispiel C: Lösung

Man bereitet eine Lösung aus 1 g 2S-[2-Oxo-3S-(H-Lys(CBZ)-D-Trp-D-Phe-amino)-pyrrolidino]-4-methyl-valeryl-Met-$NH_2$, 9,38 g $NaH_2PO_4$ . 2 $H_2O$, 28,48 g $Na_2HPO_4$ . 12 $H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

Beispiel D: Salbe

Man mischt 500 mg 2S-[2-Oxo-4-(CBZ-Asp-Phe-Phe-Val)-piperazino]-4-methyl-valeryl-Met-$NH_2$ mit 99,5 g Vaseline unter aseptischen Bedingungen.

**Ansprüche**

1. Aminosäurederivate der Formel I

$$X-Z-NR^1-CR^2R^3-CO-NR^4-CHR^5-CO-Y-R^6 \quad I$$

worin

X H, $R^7$-O-CO-, $R^7$-CO-, $R^7$-NH-CO-oder $R^8$-CO-$(CHR^7)_n$-CO-,

Z 0 bis 4 peptidartig miteinander verbundene Aminosäurereste ausgewählt aus der Gruppe bestehend aus Ala, Arg, Asn, Asp, Asp(OBut), Gln, Glu, Gly, His, Ile, Leu, Lys, Met, αNal, βNal, Orn, Phe, Pro, Ser, Thr, Tic, Trp, Tyr, Val, Cα-Me-αNal, Cα-Me-Phe, Cα-Me-Tic, Cα-Me-Trp, Cα-Me-Tyr, wobei funktionelle Gruppen der Aminosäureseitenkette auch mit einer Gruppe X geschützt sein können,

$R^1$, $R^2$, $R^3$, $R^4$, $R^5$, und $R^7$ jeweils H, A, Alkenyl oder Alkinyl mit jeweils bis zu 4 C-Atomen, Ar, Ar-alkyl, Het, Het-alkyl, unsubstituiertes oder ein-oder mehrfach durch A, AO und/oder Hal substituiertes Cycloalkyl mit 3-7 C-Atomen, Cycloalkylalkyl mit 4-11 C-Atomen,

$R^1$ und $R^4$, $R^2$ und $R^3$ bzw. $R^2$ und $R^4$ auch jeweils zusammen eine Alkylenkette mit 2-6 C-Atomen, die gesättigt oder ungesättigt, unsubstituiert oder ein-oder mehrfach durch A, OH, OA, Ar, Ar-alkyl, Het und/oder Het-alkyl substituiert sein kann, wobei $R^2$ und/oder $R^3$ nur dann H sind, wenn $R^1$ und $R^4$ bzw. $R^2$ und $R^4$ jeweils zusammen eine Alkylenkette mit 2-6 C-Atomen, die gesättigt oder ungesättigt, unsubstituiert oder ein-oder mehrfach durch A, OH, OA, Ar, Ar-alkyl und/oder Het-alkyl substituiert sein kann, bedeuten,

Y Cys, Ile, Leu, Met, Met(O), $Met(O_2)$, Nle oder Phe,

$R^6$ und $R^8$ jeweils OH, OA, $NH_2$, NHA oder $NA_2$,

n 0, 1, 2 oder 3,

Ar unsubstituiertes oder ein-oder mehrfach durch A, AO, Hal, $CF_3$, OH und/oder $NH_2$ substituiertes Phenyl oder unsubstituiertes Naphthyl,

Het einen gesättigten oder ungesättigten 5-oder 6-gliedrigen heterocyclischen Rest mit 1-4 N-, O- und/oder S-Atomen, der mit einem Benzolring oder einem Pyridinring kondensiert und/oder ein-oder mehrfach durch A, AO, Hal, $CF_3$, HO, $O_2N$, Carbonylsauerstoff, $H_2N$, HAN, $A_2N$, AcNH, AS, ASO, $ASO_2$, AOOC, CN, $H_2NCO$, ANHCO, $A_2NCO$, ArNHCO, Ar-alkyl-NHCO, $H_2NSO_2$, $ASO_2NH$, Ar, Ar-alkyl, Ar-alkenyl, Hydroxyalkyl und/oder Aminoalkyl mit jeweils 1-8 C-Atomen substituiert sein kann und/oder dessen N- und/oder S-Heteroatome auch oxydiert sein können,

-alkyl- eine Alkylenkette mit 1-4 C-Atomen,

Hal F, Cl, Br oder J,

Ac H-CO-, A-CO-, Ar-CO-, A-NH-CO-oder Ar-NH-CO-und

A Alkyl mit 1-8 C-Atomen bedeuten,

worin ferner an Stelle einer oder mehrerer -NH-CO-Gruppen auch eine oder mehrere -NA-CO-Gruppen

stehen können,

worin jedoch $-NR^1-CR^2R^3-CO-NR^4-CHR^5-CO-Y-R^6$ nur dann

(1) Aib-Phe-Met-$NH_2$ bedeutet,
wenn X-Z von BOC-GLy, von H-Tyr und von H-Tyr-Gly verschieden ist;

(2) Aib-Leu-Met-$NH_2$ bedeutet,
wenn X-Z von BOC-Phe-Phe, von BOC-Gln-Phe-Phe, von BOC-Gln-Gln-Phe-Phe und von H-Gln-Gln-Phe-Phe verschieden ist,

(3) Aib-Phe-Leu-$NH_2$ bedeutet,
wenn X-Z von H, von BOC, von H-Tyr und von BOC-Tyr-Gly verschieden ist;

(4) Aib-Phe-Leu-OMe bedeutet,
wenn X-Z von H und von BOC verschieden ist;

(5) Aib-Phe-Phe-OMe bedeutet,
wenn X-Z von CBZ verschieden ist;

(6) Aib-Trp-Phe-$NH_2$ bedeutet,
wenn X-Z von H-Tyr-Trp verschieden ist;

(7) Deg-Gly-Leu-OH bedeutet, wenn X-Z von H verschieden ist;

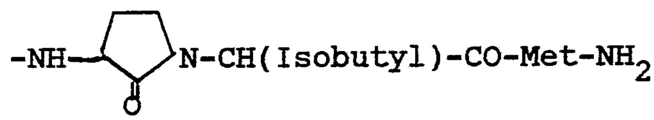

bedeutet,
wenn X-Z von H, von BOC, von Ac-Phe-Ile, von Ac-Phe-Phe, von Ac-Phe-Tyr, von Ac-Phe-Val, von Glp-Phe-Ile, von Glp-Phe-Phe, von Glp-Phe-Tyr, von Glp-Phe-Val, von H-Pro-Phe-Gly, von H-Pro-Phe-Phe, von H-Pro-Trp-Gly, von H-Pro-Trp-Phe, von BOC-Pro-Phe-Phe, von Ac-Ala-Phe-Ile, von Ac-Ala-Phe-Phe, von Ac-Ala-Phe-Tyr, von Ac-Ala-Phe-Val, von Ac-Phe-Phe-Ile, von Ac-Phe-Phe-Phe, von Ac-Phe-Phe-Tyr-und von ac-Phe-Phe-Val verschieden ist;

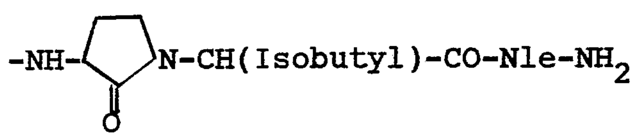

bedeutet,
wenn X-Z von H-Pro-Phe-Gly, von H-Pro-Phe-Phe, von H-Pro-Trp-Gly und von H-Pro-Trp-Phe verschieden ist;

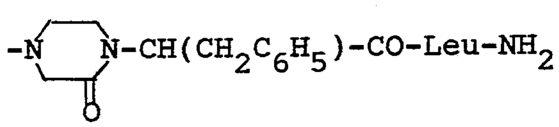

bedeutet,
wenn X-Z von H-Tyr-D-Ala verschieden ist;

(11) Cle-Phe-Leu-$NH_2$ bedeutet,
wenn X-Z von H, von BOC, von H-Tyr-Gly und von BOC-Tyr-Gly verschieden ist;

(12) Cle-Leu-Phe-OH bedeutet,
wenn X-Z von H-CO verschieden ist;

sowie deren Salze.

2.

(a) 2S-[2-Oxo-3S-(H-Lys(CBZ)-D-Trp-D-Phe-amino)-pyrrolidino]-4-methyl-valeryl-Met-$NH_2$);
(b) Glp-Phe-D-Phe-Aib-Leu-Met-$NH_2$;
(c) 2S-[2-Oxo-4-(CBZ-Asp-Phe-Phe-Val)-piperazino]-4-methyl-valeryl-Met-$NH_2$;
(d) 2S-[2-Oxo-4-(H-Asp-Phe-Phe-Val)-piperazino]-4-methyl-valeryl-Met-$NH_2$;

(e) Glp-Phe-Phe-Cle-Leu-Met-OMe;
(f) Glp-Phe-D-Phe-Cle-Leu-Met-OMe.

3. Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt

oder daß man eine Carbonsäure der Formel II

$X-G^1-OH$ II

worin $G^1$
(a) $-Z^1-$,
(b) $-Z-$,
(c) $-Z-NR^1-CR^2R^3-CO-$,
(d) $-Z-NR^1-CR^2R^3-CO-NR^4-CHR^5-CO-$,
(e) $-Z-NR^1-CR^3R^3-CO-NR^4-CHR^5-CO-Y-$
bedeutet

mit einer Aminoverbindung der Formel III

$H-G^2$ III

worin $G^2$
(a) $-Z^2-NR^1-CR^2R^3-CO-NR^4-CHR^5-CO-Y-R^6$,
(b) $-NR^1-CR^2R^3-CO-NR^4-CHR^5-CO-Y-R^6$,
(c) $-NR^4-CHR^5-CO-Y-R^6$,
(d) $-Y-R^6$,
(e) $-NH_2$, NHA oder $NA_2$ und
$Z^1 + Z^2$ zusammen Z bedeuten

umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Aminogruppe acyliert und/oder einen Rest $R^6$ und/oder $R^8$ durch Behandeln mit veresternden, solvolysierenden oder amidierenden Mitteln in einer anderen Rest $R^6$ und/oder $R^8$ umwandelt und/ oder eine Thioethergruppe zu einer Sulfoxidgruppe oder Sulfongruppe oxydiert und/oder eine Sulfoxidgruppe zu einer Thioethergruppe reduziert und/oder eine Verbindung der Formel I durch Behandeln mit einer Säure in eines ihrer Salze überführt.

4. Verfahren zur Herstellung pharmazeutischer Zubereitungen, dadurch gekennzeichnet, daß man eine Verbindung der Formel I und/oder eines ihres physiologisch unbedenklichen Salze zusammen mit mindestens einem festen, flüssigen oder halbflüssigen Träger-oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen zur Herstellung eines Arzneimittels.

7. Verwendung von Verbindungen der Formel I oder von deren physiologisch unbedenklichen Salzen bei der Bekämpfung von cardiovasculären Erkrankungen, spastischen Erkrankungen, Schmerzzuständen, Entzündungen, Erkrankungen des Zentralnervensystems und/oder des Kreislaufs und/oder bei der Stimulierung der Tränensekretion.